# EUROPEAN PATENT SPECIFICATION

(11) **EP 0 572 120 B1**
(45) Date of publication and mention of the grant of the patent: **05.07.2000**
(21) Application number: 93303291.4
(22) Date of filing: 27.04.1993
(51) Int. Cl.: C12Q 1/68, C07H 21/04

(54) **Nucleic acid probes to mycobacterium tuberculosis**
Nukleinsäuresonden für Mykobacterium Tuberkulosis
Sondes d'acides nucléiques contre du mycobacterum tuberculosis

(30) Priority: 28.04.1992 US 876283
(43) Date of publication of application: 01.12.1993
(73) Proprietor: Gen-Probe Incorporated, San Diego, CA 92121 (US)
(72) Inventor: Hammond, Philip W., San Diego, California 92116 (US)
(74) Representative: Goldin, Douglas Michael

(56) References cited:
- EP-A- 0 272 009
- EP-A- 0 318 245
- EP-A- 0 398 677
- INTERNATIONAL JOURNAL OF SYSTEMATIC BACTERIOLOGY vol. 40, no. 4, October 1990, WASHINGTON D. C. pages 323 - 330 ROGALL, T. ET AL. 'towards a phylogeny and definition of species at the molecular level within the genus mycobacterium'

## Description

### Field of the Invention

The inventions described and claimed herein relate to the design and construction of nucleic acid probes for Mycobacterium tuberculosis Complex (TB Complex) which are capable of detecting the organisms in test samples for, e.g., sputum, urine, blood and tissue sections, food, soil and water.

### Background of the Invention

Two single strands of deoxyribo- ("DNA") or ribo-("RNA") nucleic acid, formed from nucleotides (including the bases adenine (A), cytosine (C), thymidine (T), guanine (G), uracil (U), or inosine (I)), may associate ("hybridize") to form a double stranded structure in which the two strands are held together by hydrogen bonds between pairs of complementary bases. Generally, A is hydrogen bonded to T or U, while G is hydrogen bonded to C. At any point along the chain, therefore, one may find the classical base pairs AT or AU, TA or UA, GC, or CG. One may also find AG, GU and other "wobble" or mismatched base pairs.

When a first single strand of nucleic acid contains sufficient contiguous complementary bases to a second, and those two strands are brought together under conditions which will promote their hybridization, double stranded nucleic acid will result. Under appropriate conditions, DNA/DNA, RNA/DNA, or RNA/RNA hybrids may be formed.

A probe is generally a single stranded nucleic acid sequence which is complementary to some degree to a nucleic acid sequence sought to be detected ("target sequence"). It may be labelled with a detectable moiety such as a radioisotope, antigen or chemiluminescent moiety. A background description of the use of nucleic acid hybridization as a procedure for the detection of particular nucleic acid sequences is provided by U.S. Patent No. 4,851,330, and EP-A 0 272 009 entitled "Nucleic Acid Probes for Detection and/Or Quantitation of Non-viral Organisms."

EP-A 0 272 009 also describes methods for determining the presence of RNA-containing organisms in a sample which might contain such organisms. These methods require probes sufficiently complementary to hybridize to the ribosomal RNA (rRNA) of one or more non-viral organisms or groups of non-viral organisms. The mixture is then incubated under specified hybridization conditions, and assayed for hybridization of the probe and any test sample rRNA.

EP-A 0 272 009 additionally describes probes which detect only specifically targeted rRNA subunit subsequences in particular organisms or groups of organisms in a sample, even in the presence of many non-related organisms, or in the presence of the closest known phylogenetic neighbors. Specific examples of hybridization assay probes are provided for Mycobacterium tuberculosis. Such probe sequences do not cross react with nucleic acids from other bacterial species or infectious agent, under appropriate hybridization stringency conditions.

### Summary of the Invention

This invention discloses and claims novel probes for the detection of Mycobacterium tuberculosis (TB) Complex. These probes are capable of distinguishing between the Mycobacterium tuberculosis Complex and its known closest phylogenetic neighbors. The Mycobacterium tuberculosis Complex consists of the following species: M. tuberculosis, M. bovis, M. bovis BCG, M. africanum, M. microti. These probes detect unique rRNA and gene sequences encoding rRNA, and may be used in an assay for the detection and/or quantitation of Mycobacterium tuberculosis Complex.

Organisms of the TB Complex are responsible for significant morbidity and mortality in humans. M. tuberculosis is the most common TB Complex pathogen isolated from humans. M. bovis BCG may be transmitted from infected animals to humans. M. africanum causes pulmonary tuberculosis in tropical Africa and M. microti primarily infects animals.

Tuberculosis is highly contagious, therefore rapid diagnosis of the disease is important. For most clinical laboratories assignment of an isolate to the TB Complex is sufficient because the probability that an isolate is a species other than M. tuberculosis is extremely small. A number of biochemical tests are recommended to speciate members of the TB Complex if further differentiation is required.

Classical methods for identification of mycobacteria rely on staining specimens for acid fast bacilli followed by culture and biochemical testing. It could take as long as two months to speciate an isolate using these standard methods. The use of DNA probes of this invention identifies TB Complex isolated from culture in less than an hour.

Thus, in a first aspect, the invention features a hybridization assay probe able to distinguish Mycobacterium tuberculosis from other Mycobacterium species; specifically, the probe is an oligonucleotide which hybridizes to the rRNA of the species Mycobacterium tuberculosis at a location corresponding to 23 bases in the insert region beginning at the equivalent of base 270 of E. coli 23S rRNA, or to 21 bases in the insert region beginning at the equivalent of base 1415 of E. coli 23S rRNA, or an oligonucleotide complementary thereto; that is, the oligonucleotide comprises, consists essentially of, or consists of the sequence
(SEQ ID NO: 1) GGTAGCGCTGAGACATATCCTCC, or (SEQ ID NO: 2) CAGAACTCCACACCCCCGAAG, or oligonucleotides complementary thereto, with or without a helper probe, as described below.

By "consists essentially of" is meant that the probe is provided as a purified nucleic acid which hybridizes under stringent hybridizing conditions with the desired organism and not with other related organisms. Such a probe may be linked to other nucleic acids which do not affect such hybridization. Generally, it is preferred that the probe be of between 15 and 100 (most preferably between 20 and 50) bases in size. It may, however, be provided in a vector.

In related aspects, the invention features a nucleotide polymer able to hybridize to the above oligonucleotides, a nucleic acid hybrid formed with the above oligonucleotides, and a nucleic acid sequence substantially complementary thereto. Such hybrids are useful since they allow specific detection of the TB complex organisms.

The probes of this invention offer a rapid, non-subjective method of identification and quantitation of a bacterial colony for the presence of specific rRNA sequences unique to all species and strains of Mycobacterium tuberculosis Complex.

Other features and advantages of the invention will be apparent from the following description of the preferred embodiments thereof, and from the claims.

### Description of the Preferred Embodiments

### Probes

We have discovered DNA probes complementary to a particular rRNA sequence obtained from Mycobacterium tuberculosis. Furthermore, we have successfully used those probes in a specific assay for the detection of Mycobacterium tuberculosis, distinguishing members of the M. tuberculosis complex from their known and presumably most closely related taxonomic or phylogenetic neighbors.

We have identified suitable variable regions of the target nucleic acid by comparative analysis of rRNA sequences both published in the literature and sequences which we have determined. Computers and computer programs which may be used or adapted for the purposes herein disclosed are commercially available. Since the sequence evolution at each of the variable regions (for example, spanning a minimum of 10 nucleotides) is, for the most part, divergent, not convergent, we can confidently design probes based on a few rRNA sequences which differ between the target organism and its phylogenetically closest relatives. We have seen sufficient variation between the target organism and the closest phylogenetic relative found in the same sample to design the probe of interest.

We have identified the following useful guidelines for designing probes with desired characteristics. Because the extent and specificity of hybridization reactions such as those described herein are affected by a number of factors, manipulation of one or more of those factors will determine the exact sensitivity and specificity of a particular probe, whether perfectly complementary to its target or not. The importance and effect of various assay conditions, explained further herein, are known to those skilled in the art.

First, the stability of the probe:target nucleic acid hybrid should be chosen to be compatible with the assay conditions. This may be accomplished by avoiding long A and T rich sequences, by terminating the hybrids with G:C base pairs, and by designing the probe with an appropriate Tm. The beginning and end points of the probe should be chosen so that the length and %G and %C result in a Tm about 2-10°C higher than the temperature at which the final assay will be performed. The base composition of the probe is significant because G-C base pairs exhibit greater thermal stability as compared to A-T base pairs due to additional hydrogen bonding. Thus, hybridization involving complementary nucleic acids of higher G-C content will be stable at higher temperatures.

Conditions such as ionic strength and incubation temperature under which a probe will be used should also be taken into account in constructing a probe. It is known that hybridization will increase as the ionic strength of the reaction mixture increases, and that the thermal stability of hybrids will increase with increasing ionic strength. On the other hand, chemical reagents, such as formamide, urea, DMSO and alcohols, which disrupt hydrogen bonds, will increase the stringency of hybridization. Destabilization of the hydrogen bonds by such reagents can greatly reduce the Tm. In general, optimal hybridization for synthetic oligonucleotide probes of about 10-50 bases in length occurs approximately 5°C below the melting temperature for a given duplex. Incubation at temperatures below the optimum may allow mismatched base sequences to hybridize and can therefore result in reduced specificity.

It is desirable to have probes which hybridize only under conditions of high stringency. Under high stringency conditions only highly complementary nucleic acid hybrids will form (i.e., those having at least about 14 out of 17 bases in a contiguous series of bases being complementary); hybrids without a sufficient degree of complementarity will not form. Accordingly, the stringency of the assay conditions determines the amount of complementarity needed between two nucleic acid strands forming a hybrid. Stringency is chosen to maximize the difference in stability between the hybrid formed with the target and the nontarget nucleic acid.

Second, probes should be positioned so as to minimize the stability of the probe:nontarget nucleic acid hybrid. This may be accomplished by minimizing the length of perfect complementarity to non-target organisms, avoiding G and C rich regions of homology to non-target sequences, and by positioning the probe to span as many destabilizing mismatches as possible. Whether a probe sequence is useful to detect only a specific type of organism depends largely on the thermal stability difference between probe:target hybrids and probe:nontarget hybrids. In designing probes, the differences in these Tm values should be as large as possible (e.g., at least 2°C and preferably 5°C).

The length of the target nucleic acid sequence and, accordingly, the length of the probe sequence can also be important. In some cases, there may be several sequences from a particular region, varying in location and length, which will yield probes with the desired hybridization characteristics. In other cases, one sequence may be significantly better than another which differs merely by a single base. While it is possible for nucleic acids that are not perfectly complementary to hybridize, the longest stretch of perfectly homologous base sequence will normally primarily determine hybrid stability. While oligonucleotide probes of different lengths and base composition may be used, oligonucleotide probes preferred in this invention are between about 10 to 50 bases in length and are sufficiently homologous to the target nucleic acid.

Third, regions of the rRNA which are known to form strong internal structures inhibitory to hybridization are less preferred. Likewise, probes with extensive self-complementarity should be avoided.

As explained above, hybridization is the association of two single strands of complementary nucleic acid to form a hydrogen bonded double strand. It is implicit that if one of the two strands is wholly or partially involved in a hybrid that it will be less able to participate in formation of a new hybrid. In the case of rRNA, the molecule is known to form very stable intra-molecular hybrids. By designing a probe so that a substantial portion of the sequence of interest is single stranded, the rate and extent of hybridization may be greatly increased. If the target is the genomic sequence corresponding to the rRNA then it will naturally occur in a double stranded form, this is also the case with the product of the polymerase chain reaction (PCR). These double stranded targets are naturally inhibitory to hybridization with a probe. Finally, there can be intramolecular and intermolecular hybrids formed within a probe if there is sufficient self complementarity. Such structures can be avoided through careful probe design. Computer programs are available to search for this type of interaction.

Once a presumptive unique sequence has been identified, a complementary DNA oligonucleotide is produced. This single stranded oligonucleotide will serve as the probe in the hybridization reaction. Defined oligo-nucleotides may be produced by any of several well known methods, including automated solid-phase chemical synthesis using cyanoethylphosphoramidite precursors. Barone et al., 12 Nucleic Acids Research 4051, 1984. Other well-known methods for construction of synthetic oligonucleotides may, of course, be employed. Sambrook et al., 2 Molecular Cloning 11 (2d ed. 1989).

Once synthesized, selected oligonucleotide probes may also be labelled by any of several well known methods. Sambrook et al., supra. Useful labels include radio-isotopes as well as non-radioactive reporting groups. Isotopic labels include ³H, ³⁵S, ³²P, ¹²⁵I, Cobalt and ¹⁴C. Most methods of isotopic labelling involve the use of enzymes and include the known methods of nick translation, end labelling, second strand synthesis, and reverse transcription. When using radio-labelled probes, hybridization can be detected by autoradiography, scintillation counting, or gamma counting. The detection method selected will depend upon the hybridization conditions and the particular radio-isotope used for labelling.

Non-isotopic materials can also be used for labelling, and may be introduced internally into the sequence or at the end of the sequence. Modified nucleotides may be incorporated enzymatically or chemically and chemical modifications of the probe may be performed during or after synthesis of the probe, for example, by the use of non-nucleotide linker groups. Non-isotopic labels include fluorescent molecules, chemiluminescent molecules, enzymes, cofactors, enzyme substrates, haptens or other ligands. We currently prefer to use acridinium esters.

Following synthesis and purification of a particular oligonucleotide sequence, several procedures may be utilized to determine the acceptability of the final product. The first is polyacrylamide gel electrophoresis, which is used to determine size. Sambrook et al., supra. Such procedures are known in the art. In addition to polyacrylamide gel electrophoresis, High Pressure Liquid Chromatography ("HPLC") procedures also may be used to determine the size and purity of the oligonucleotide product. These procedures are also known to those skilled in the art.

It will be appreciated by those skilled in the art that factors which affect the thermal stability can affect probe specificity and therefore, must be controlled. Thus, the melting profile, including the melting temperature (Tm) of the oligonucleotide/target hybrids should be determined. The preferred method is described in Arnold et al., WO8902476, filed September 21, 1988, entitled "Homogeneous Protection Assay,".

For Tm measurement using a Hybridization Protection Assay (HPA) the following technique is used. A probe:target hybrid is formed in target excess in a lithium succinate buffered solution containing lithium lauryl sulfate. Aliquots of this hybrid are diluted in the hybridization buffer and incubated for five minutes at various temperatures starting below that of the anticipated Tm (typically 55°C) and increasing in 2-5 degree increments. This solution is then diluted with a mildly alkaline borate buffer and incubated at a lower temperature (for example 50°C) for ten minutes. Under these conditions the acridinium ester attached to a single stranded probe is hydrolyzed while that attached to hybridized probe is relatively protected from hydrolysis. The amount of chemiluminescence remaining is proportional to the amount of hybrid, and is measured in a luminometer by addition of hydrogen peroxide followed by alkali. The data is plotted as percent of maximum signal (usually from the lowest temperature) versus temperature. The Tm is defined as the point at which 50% of the maximum signal remains.

In addition to the above method, oligonucleotide/target hybrid melting temperature may also be determined by isotopic methods well known to those skilled in the art. It should be noted that the Tm for a given hybrid will vary depending on the hybridization solution being used because the thermal stability depends upon the concentration of different salts, detergents, and other solutes which effect relative hybrid stability during thermal denaturation. Sambrook et al., supra.

Rate of hybridization may be measured by determining the C₀t_{½}. The rate at which a probe hybridizes to its target is a measure of the thermal stability of the target secondary structure in the probe region. The standard measurement of hybridization rate is the C₀t_{½} which is measured as moles of nucleotide per liter times seconds. Thus, it is the concentration of probe times the half-life of hybridization at that concentration. This value is determined by hybridizing various amounts of probe to a constant amount of hybrid for a fixed time. For example, 0.05 pmol of target is incubated with 0.0012, 0.025, 0.05, 0.1 and 0.2 pmol of probe for 30 minutes. The amount of hybrid after 30 minutes is measured by HPA as described above. The signal is then plotted as a log of the percent of maximum Relative Light Units (RLU) (from the highest probe concentration) versus probe concentration (moles of nucleotide per liter). RLU are a measurement of the quantity of photons emitted by the labelled-probe measured by the luminometer. The C₀t_{½} is found graphically from the concentration corresponding to 50% of maximum hybridization multiplied by the hybridization time in seconds. These values range from 9.0x10⁻⁶ to 9x10⁻⁵ with the preferred values being less than 3.5x10⁻⁵.

As described by Kohne and Kacian EP 229442, filed June 10, 1986), other methods of nucleic acid reassociation can be used.

The following example sets forth synthetic probes complementary to a unique rRNA sequence, or the corresponding gene, from a target organism, Mycobacterium tuberculosis, and their use in a hybridization assay.

### Example:

A probe specific for M. tuberculosis was identified by sequencing with a primer complementary to the 16S rRNA. The following sequences were characterized and shown to be specific for Mycobacterium tuberculosis; (SEQ ID NO: 1) GGTAGCGCTGAGACATATCCTCC, and (SEQ ID NO: 2) CAGAACTCCACACCCCCGAAG. Several phylogenetically near neighbors including M. kansasii, M. asiaticum and M. avium were used as comparisons with the sequence of M. tuberculosis. SEQ ID NO: 1 is 23 bases in length and hybridizes to the 23S rRNA of M. tuberculosis corresponding to bases 270-293 of E. coli. SEQ ID NO: 2 is 21 bases in length and hybridizes to the 23S rRNA of M. tuberculosis corresponding to bases 1415-1436 of E. coli.

To demonstrate the reactivity and specificity of the probe for M. tuberculosis, it was used in a hybridization assay. The probe was first synthesized with a non-nucleotide linker, then labelled with a chemiluminescent acridinium ester as described in EPO Patent Application No. EP 312248, entitled "Acridinium Ester Labeling and Purification of Nucleotide Probes filed October 5, 1988. The acridinium ester attached to unhybridized probe is rendered non-chemiluminescent under mild alkaline conditions, while the acridinium ester attached to hybridized probe is relatively resistant. Thus, it is possible to assay for hybridization of acridinium ester-labelled probe by incubation with an alkaline buffer, followed by detection of chemiluminescence in a luminometer. Results are given in RLU, the quantity of photons emitted by the labelled-probe measured by the luminometer. The conditions of hybridization, hydrolysis and detection are described in Arnold, et al., 35 Clin. Chem. 1588, 1989.

Nucleic acid hybridization was enhanced by the use of "Helper Probes" as disclosed in Hogan et al., U.S. Patent No. 5,030,557 RNA was hybridized to the acridinium ester-labeled probe in the presence of an unlabeled Helper Probe. The probe corresponding to oligonucleotide SEQ ID NO: 1 with helpers:
(SEQ ID NO: 3) CCGCTAACCACGACACTTTCTGTACTGCCTCTCAGCCG and
(SEQ ID NO: 4) CACAACCCCGCACACACAACCCCTACCCGGTTACCC.
The probe corresponding to oligonucleotide SEQ ID NO: 2 with helpers: (SEQ ID NO: 5) TGATTCGTCACGGGCGCCCACACACGGGTACGGGAATATCAACCC and
(SEQ ID NO: 6) CTACTACCAGCCGAAGTTCCCACGCAGCCC and
(SEQ ID NO: 7) GGAGTTGATCGATCCGGTTTTGGGTGGTTAGTACCGC and
(SEQ ID NO: 8) GGGGTACGGGCCGTGTGTGTGCTCGCTAGAGGCTTTTCTTGGC.

In the following experiment, RNA released from one colony or >10⁸ organisms was assayed. An example of such a method is provided by Murphy et al. (EP 873036412, filed April 24, 1987 and published as EP-A 0 288618. An RLU value greater than 30,000 RLU is a positive reaction; less than 30,000 is a negative reaction.

The following data show that the probes did not cross react with organisms from a wide phylogenetic cross section. The samples were also tested with a Probe (ALL BACT.) which has a very broad specificity to provide a positive control. A positive signal from this probe provides confirmation of sample adequacy.

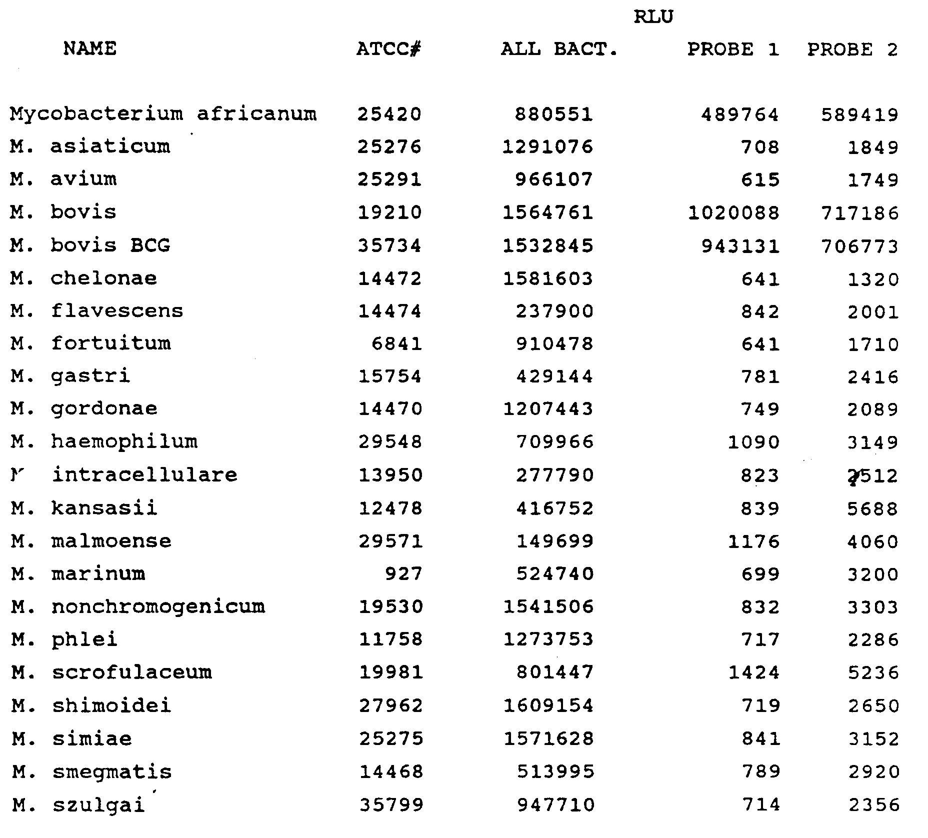

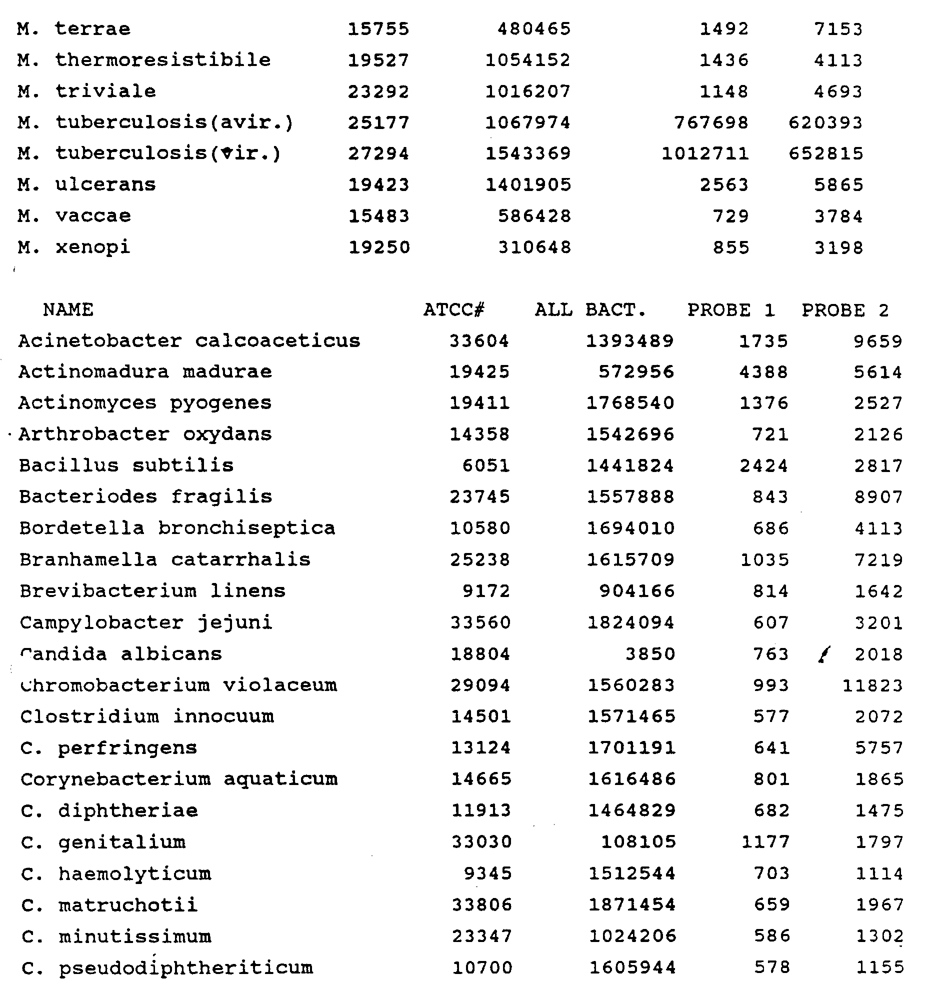

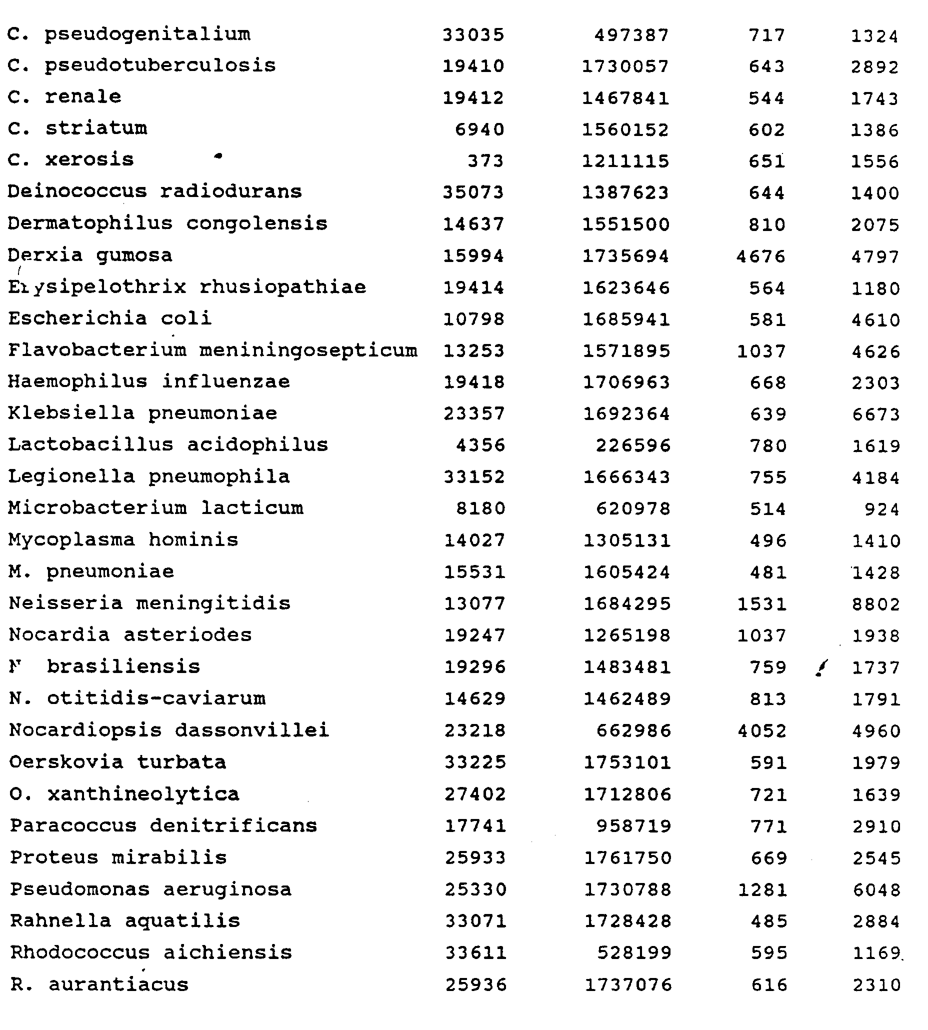

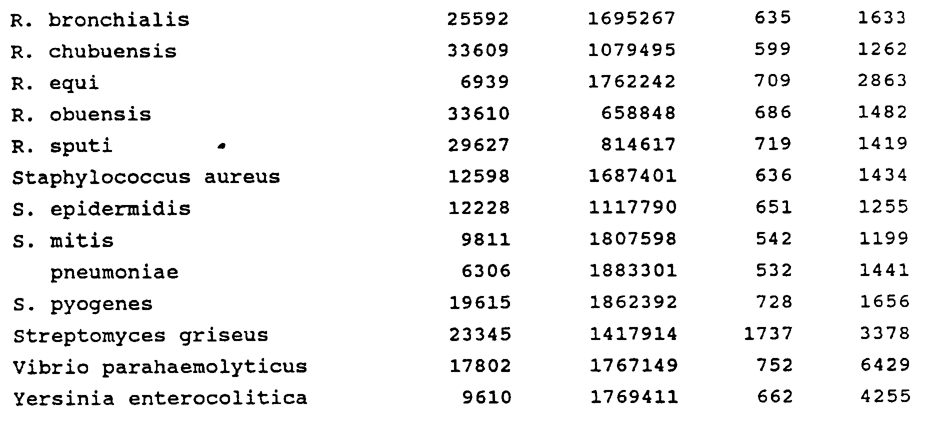

The above data confirm that the novel probes herein disclosed and claimed are capable of distinguishing members of the Mycobacterium tuberculosis complex from their known nearest phylogenetic neighbors.

Other embodiments are within the following claims.

## Claims

1. A hybridization assay probe able to detect the presence of *Mycobacterium tuberculosis*, *Mycobacterium bovis*, *Mycobacterium bovis BCG* and *Mycobacterium africanum*, comprising a purified oligonucleotide containing at least 14 out of 17 contiguous bases perfectly complementary to a nucleic acid sequence selected from GGTAGCGCTGAGACATATCCTCC (Sequence ID no 1) and the sequence complementary thereto;
wherein under high stringency hybridization conditions said probe hybridizes to nucleic acid from *Mycobacterium tuberculosis*, *Mycobacterium bovis*, *Mycobacterium bovis BCG* and *Mycobacterium africanum* to form a detectable probe: target duplex, and
wherein under said conditions said probe distinguishes *Mycobacterium tuberculosis*, *Mycobacterium bovis*, *Mycobacterium bovis BCG* and *Mycobacterium africanum* nucleic acid from *Mycobacterium avium*, *Mycobacterium asiaticum*, and *Mycobacterium kansasii* nucleic acid.

2. A probe as claimed in claim 1, wherein said oligonucleotide comprises a nucleotide sequence selected from GGTAGCGCTGAGACATATCCTCC and the sequence complementary thereto.

3. A probe as claimed in claim 2, wherein said oligonucleotide consists of a nucleotide sequence selected from GGTAGCGCTGAGACATATCCTCC and the sequence complementary thereto.

4. A probe as claimed in claim 1 or claim 2, wherein said oligonucleotide is between 20 and 50 bases in length.

5. A hybridization assay probe able to detect the presence of *Mycobacterium tuberculosis*, *Mycobacterium bovis*, *Mycobacterium bovis BCG* and *Mycobacterium africanum*, comprising a purified oligonucleotide containing at least 14 out of 17 contiguous bases perfectly complementary to a nucleic acid sequence selected from CAGAACTCCACACCCCCGAAG (Sequence ID no 2) and the sequence complementary thereto;
wherein under high stringency hybridization conditions said probe hybridizes to nucleic acid from *Mycobacterium tuberculosis*, *Mycobacterium bovis*, *Mycobacterium bovis BCG* and *Mycobacterium africanum* nucleic acid to form a detectable probe:target duplex; and
wherein under said conditions said probe distinguishes *Mycobacterium tuberculosis*, *Mycobacterium bovis*, *Mycobacterium bovis BCG* and *Mycobacterium africanum* nucleic acid from *Mycobacterium avium*, *Mycobacterium asiaticum*, and *Mycobacterium kansasii* nucleic acid.

6. A probe as claimed in claim 5, wherein said oligonucleotide comprises a nucleotide sequence selected from CAGAACTCCACACCCCCGAAG and the sequence complementary thereto.

7. A probe as claimed in claim 6, wherein said oligonucleotide consists of a nucleotide sequence selected from CAGAACTCCACACCCCCGAAG and the sequence complementary thereto.

8. A probe as claimed in claim 5 or claim 6, wherein said oligonucleotide is between 20 and 50 bases in length.

9. A nucleic acid hybrid formed between an oligonucleotide as claimed in any one of claims 1 to 8 and a nucleic acid complementary thereto.

10. A probe mix comprising an oligonucleotide as claimed in any one of claims 1 to 4 and a helper probe.

11. A probe mix as claimed in claim 10, wherein said helper probe is an oligonucleotide comprising the oligonucleotide sequence shown as SEQ ID NOS: 3 or 4 or a sequence complementary thereto.

12. A probe mix comprising an oligonucleotide as claimed in any one of claims 5 to 8 and a helper probe.

13. A probe mix as claimed in claim 12, wherein said helper probe is an oligonucleotide comprising the oligonucleotide sequence shown as SEQ ID NO: 5, 6, 7 or 8, or a sequence complementary thereto.

14. A method for determining whether *Mycobacterium tuberculosis*, *Mycobacterium bovis*, *Mycobacterium bovis BCG* or *Mycobacterium africanum* may be present in a sample comprising the steps of:
a) contacting said sample with a hybridization assay probe as claimed in any one of claims 1 to 8 under high stringency hybridization conditions; and
b) detecting the presence of probe:target duplex formed under said hybridization conditions as an indication that *Mycobacterium tuberculosis*, *Mycobacterium bovis*, *Mycobacterium bovis BCG* or *Mycobacterium africanum* may be present in said sample.

15. A method for determining whether *Mycobacterium tuberculosis*, *Mycobacterium bovis*, *Mycobacterium bovis BCG* or *Mycobacterium africanum* may be present in a sample comprising the steps:
a) contacting said sample with a probe mix as claimed in any one of claims 10 to 13 under high stringency hybridization conditions; and
b) detecting the presence of hybridization probe:target duplex formed under said hybridization conditions as an indication that *Mycobacterium tuberculosis*, *Mycobacterium bovis*, *Mycobacterium bovis BCG* or *Mycobacterium africanum* may be present in said sample.

## Patentansprüche

1. Hybridisierungsassay-Sonde, mit der der Nachweis des Vorhandenseins von *Mycobacterium tuberculosis*, *Mycobacterium bovis*, *Mycobacterium bovis BCG* und *Mycobacterium africanum* möglich ist, umfassend ein gereinigtes Oligonukleotid, das mindestens 14 von 17 benachbarten Basen enthält, die perfekt komplementär zu einer Nukleinsäure-Sequenz sind, gewählt aus GGTAGCGCTGAGACATATCCTCC (Sequenz ID Nr. 1) und der dazu komplementären Sequenz;
wobei unter hochstringenten Hybridisierungsbedingungen die Sonde an Nukleinsäure von *Mycobacterium tuberculosis*, *Mycobacterium bovis*, *Mycobacterium bovis BCG* und *Mycobacterium africanum* hybridisiert und dabei einen nachweisbaren Sonden:Ziel-Duplex bildet, und
wobei unter diesen Bedingungen diese Sonde Nukleinsäure von *Mycobacterium tuberculosis*, *Mycobacterium bovis*, *Mycobacterium bovis BCG* und *Mycobacterium africanum* von Nukleinsäure von *Mycobacterium avium*, *Mycobacterium asiaticum* und *Mycobacterium kansasii* unterscheidet.

2. Sonde nach Anspruch 1, wobei das Oligonukleotid eine Nukleotidsequenz enthält, gewählt aus GGTAGCGCTGAGACATATCCTCC und der dazu komplementären Sequenz.

3. Sonde nach Anspruch 2, wobei das Oligonukleotid in einer Nukleotidsequenz besteht, gewählt aus GGTAGCGCTGAGACATATCCTCC und der dazu komplementären Sequenz.

4. Sonde nach Anspruch 1 oder Anspruch 2, wobei das Oligonukleotid zwischen 20 und 50 Basen lang ist.

5. Hybridisierungsassay-Sonde, mit der der Nachweis des Vorhandenseins von *Mycobacterium tuberculosis*, *Mycobacterium bovis*, *Mycobacterium bovis BCG* und *Mycobacterium africanum* möglich ist, umfassend ein gereinigtes Oligonukleotid, die mindestens 14 von 17 benachbarten Basen enthält, die perfekt komplementär zu einer Nukleinsäure-Sequenz sind, gewählt aus CAGAACTCCACACCCCCGAAG (Sequenz ID Nr. 2) und der dazu komplementären Sequenz;
wobei unter hochstringenten Hybridisierungsbedingungen die Sonde an Nukleinsäure von *Mycobacterium tuberculosis*, *Mycobacterium bovis*, *Mycobacterium bovis BCG* und *Mycobacterium africanum* hybridisiert und dabei einen nachweisbaren Sonden:Ziel-Duplex bildet, und
wobei unter diesen Bedingungen diese Sonde Nukleinsäure von *Mycobacterium tuberculosis*, *Mycobacterium bovis*, *Mycobacterium bovis BCG* und *Mycobacterium africanum* von Nukleinsäure von *Mycobacterium avium*, *Mycobacterium asiaticum* und *Mycobacterium kansasii* unterscheidet.

6. Sonde nach Anspruch 5, wobei das Oligonukleotid eine Nukleotidsequenz enthält, gewählt aus CAGAACTCCACACCCCCGAAG und der dazu komplementären Sequenz.

7. Sonde nach Anspruch 6, wobei das Oligonukleotid in einer Nukleotidsequenz besteht, gewählt aus CAGAACTCCACACCCCCGAAG und der dazu komplementären Sequenz.

8. Sonde nach Anspruch 5 oder Anspruch 6, wobei das Oligonukleotid zwischen 20 und 50 Basen lang ist.

9. Nukleinsäure-Hybrid, gebildet zwischen einem Oligonukleotid nach einem der Ansprüche 1 bis 8 und einer dazu komplementären Nukleinsäure.

10. Sondengemisch, umfassend ein Oligonukleotid nach einem der Ansprüche 1 bis 4 und eine Helfer-Sonde.

11. Sondengemisch nach Anspruch 10, wobei die Helfer-Sonde ein Oligonukleotid ist, umfassend die als SEQ ID NRN. 3 oder 4 gezeigte Oligonukleotid-Sequenz oder eine dazu komplementäre Sequenz.

12. Sondengemisch, umfassend ein Oligonukleotid nach einem der Ansprüche 5 bis 8 und eine Helfer-Sonde.

13. Sondengemisch nach Anspruch 12, wobei die Helfer-Sonde ein Oligonukleotid ist, umfassend die als SEQ ID NR. 5, 6, 7 oder 8 gezeigte Oligonukleotid-Sequenz oder eine dazu komplementäre Sequenz.

14. Verfahren zur Bestimmung des möglichen Vorhandenseins von *Mycobacterium tuberculosis*, *Mycobacterium bovis*, *Mycobacterium bovis BCG* und *Mycobacterium africanum* in einer Probe, umfassend die folgenden Schritte:
a) Zusammenbringen dieser Probe mit einer Hybridisierungsassay-Sonde nach einem der Ansprüche 1 bis 8 unter hochstringenten Hybridisierungsbedingungen; und
b) Nachweisen des Vorhandenseins von Sonden:Ziel-Duplex, wie unter diesen Hybridisierungsbedingungen gebildet, als einen Hinweis auf das mögliche Vorhandensein von *Mycobacterium tuberculosis*, *Mycobacterium* *bovis*, *Mycobacterium bovis BCG* oder *Mycobacterium africanum* in der Probe.

15. Verfahren zur Bestimmung des möglichen Vorhandenseins von *Mycobacterium tuberculosis*, *Mycobacterium bovis*, *Mycobacterium bovis BCG* und *Mycobacterium africanum* in einer Probe, umfassend die folgenden Schritte:
a) Zusammenbringen dieser Probe mit einem Sondengemisch nach einem der Ansprüche 10 bis 13 unter hochstringenten Hybridisierungsbedingungen; und
b) Nachweisen des Vorhandenseins des Hybridisierungsonden:Ziel-Duplex, wie unter diesen Hybridisierungsbedingungen gebildet, als einen Hinweis auf das mögliche Vorhandensein von *Mycobacterium tuberculosis*, *Mycobacterium bovis*, *Mycobacterium bovis BCG* oder *Mycobacterium africanum* in der Probe.

## Revendications

1. Sonde de test d'hybridation capable de détecter la présence de *Mycobacterium tuberculosis*, *Mycobacterium bovis*, *Mycobacterium bovis BCG* et *Mycobacterium africanum*, comprenant un oligonucléotide purifié contenant au moins 14 sur 17 bases contiguës parfaitement complémentaires d'une séquence d'acide nucléique choisie parmi GGTAGCGCTGAGACATATCCTCC (Séquence ID n° 1) et la séquence complémentaire de celle-ci ;
ladite sonde s'hybridant dans des conditions d'hybridation fortement stringentes, avec un acide nucléique de *Mycobacterium tuberculosis*, *Mycobacterium bovis*, *Mycabacterium bovis BCG* et *Mycobacterium africanum* pour former un duplex sonde : cible détectable, et
ladite sonde distingant, dans lesdites conditions, un acide nucléique de *Mycobacterium tuberculosis*, *Mycobacterium bovis*, *Mycobacterium bovis BCG* et *Mycobacterium africanum* d'un acide nucléique de *Mycobacterium avium*, *Mycobacterium asiaticum*, et *Mycobacterium kansasii*.

2. Sonde telle que revendiquée à la revendication 1, dans laquelle ledit oligonucléotide comprend une séquence nucléotidique choisie parmi GGTAGCGCTGAGACATATCCTCC et la séquence complémentaire de celle-ci.

3. Sonde telle que revendiquée à la revendication 2, dans laquelle ledit oligonucléotide est constitué d'une séquence nucléotidique choisie parmi GGTAGCGCTGAGACATATCCTCC et la séquence complémentaire de celle-ci.

4. Sonde telle que revendiquée à la revendication 1 ou à la revendication 2, dans laquelle ledit oligonucléotide a une longueur comprise entre 20 et 50 bases.

5. Sonde de test d'hybridation capable de détecter la présence de *Mycobacterium tuberculosis*, *Mycobacterium bovis*, *Mycobacterium bovis BCG* et *Mycobacterium africanum*, comprenant un oligonucléotide purifié contenant au moins 14 sur 17 bases contiguës parfaitement complémentaires d'une séquence d'acide nucléique choisie parmi CAGAACTCCACACCCCCGAAG (Séquence ID n° 2) et la séquence complémentaire de celle-ci ;
ladite sonde s'hybridant, dans des conditions d'hybridation fortement stringentes, avec un acide nucléique de *Mycobacterium tuberculosis*, *Mycobacterium bovis*, *Mycobacterium* bovis *BCG* et *Mycobacterium africanum* pour former un duplex sonde : cible détectable ; et
ladite sonde distingant, dans lesdites conditions, un acide nucléique de *Mycobacterium tuberculosis*, *Mycobacterium bovis*, *Mycobacterium bovis BCG* et *Mycobacterium africanum* d'un acide nucléique de *Mycobacterium avium*, *Mycobacterium asiaticum*, et *Mycobacterium kansasii.*

6. Sonde telle que revendiquée à la revendication 5, dans laquelle ledit oligonucléotide comprend une séquence nucléotidique choisie parmi CAGAACTCCACACCCCCGAAG et la séquence complémentaire de celle-ci.

7. Sonde telle que revendiquée à la revendication 6, dans laquelle ledit oligonucléotide est constitué d'une séquence nucléotidique choisie parmi CAGAACTCCACACCCCCGAAG et la séquence complémentaire de celle-ci.

8. Sonde telle que revendiquée à la revendication 5 ou à la revendication 6, dans laquelle ledit oligonucléotide a une longueur comprise entre 20 et 50 bases.

9. Hybride d'acide nucléique formé entre un oligonucléotide tel que revendiqué dans l'une quelconque des revendications 1 à 8 et un acide nucléique complémentaire de celui-ci.

10. Mélange de sondes comprenant un oligonucléotide tel que revendiqué dans l'une quelconque des revendications 1 à 4 et une sonde auxiliaire.

11. Mélange de sondes tel que revendiqué à la revendication 10, dans lequel ladite sonde auxiliaire est un oligonucléotide comprenant la séquence oligonucléotidique présentée comme SEQ ID n° 3 ou 4 ou une séquence complémentaire de celles-ci.

12. Mélange de sondes comprenant un oligonucléotide tel que revendiqué dans l'une quelconque des revendications 5 à 8 et une sonde auxiliaire.

13. Mélange de sondes tel que revendiqué à la revendication 12, dans lequel ladite sonde auxiliaire est un oligonucléotide comprenant la séquence oligonucléotidique présentée conune SEQ ID n° 5, 6, 7 ou 8 ou une séquence complémentaire de celles-ci.

14. Méthode pour déterminer si *Mycobacterium tuberculosis*, *Mycobacterium bovis*, *Mycobacterium bovis BCG* ou *Mycobacterium africanum* peut être présent dans un échantillon comprenant les étapes consistant :
a) à mettre ledit échantillon en contact avec une sonde de test d'hybridation telle que revendiquée dans l'une quelconque des revendications 1 à 8 dans des conditions d'hybridation fortement stringentes ; et
b) à détecter la présence de duplex sonde : cible formé dans lesdites conditions d'hybridation comme une indication que *Mycobacterium tuberculosis*, *Mycobacterium bovis*, *Mycobacterium bovis BCG* ou *Mycobacterium africanum* peut être présent dans ledit échantillon.

15. Méthode pour déterminer si *Mycobacterium tuberculosis*, *Mycobacterium bovis*, *Mycobacterium bovis BCG* ou *Mycobacterium africanum* peut être présent dans un échantillon comprenant les étapes consistant :
a) à mettre ledit échantillon en contact avec un mélange de sondes tel que revendiqué dans l'une quelconque des revendications 10 à 13 dans des conditions d'hybridation fortement stringentes ; et
b) à détecter la présence de duplex d'hybridation sonde : cible formé dans lesdites conditions d'hybridation comme une indication que *Mycobacterium tuberculosis*, Myco*bacterium bovis*, *Mycobacterium bovis BCG* ou *Mycobacterium africanum* peut être présent dans ledit échantillon.


(1) GENERAL INFORMATION:

(i) APPLICANT:                GEN-PROBE INCORPORATED
                              9880 CAMPUS POINT DRIVE,
                              SAN DIEGO, CALIFORNIA 92121, U.S.A

(ii) TITLE OF INVENTION:    NUCLEIC ACID PROBES TO
          MYCOBACTERIUM TUBERCULOSIS

(iii) NUMBER OF SEQUENCES:  8

(2) INFORMATION FOR SEQ ID NO: 1:

(i) SEQUENCE CHARACTERISTICS:

(A) LENGTH: 23
(B) TYPE: nucleic acid
(C) STRANDEDNESS: single
(D) TOPOLOGY: linear

(ii) SEQUENCE DESCRIPTION : SEQ ID NO: 1:

GGTAGCGCTG AGACATATCC TCC 23

(3) INFORMATION FOR SEQ ID NO: 2:

(i) SEQUENCE CHARACTERISTICS:

(A) LENGTH: 21
(B) TYPE: nucleic acid
(C) STRANDEDNESS: single
(D) TOPOLOGY: linear

(ii) SEQUENCE DESCRIPTION : SEQ ID NO: 2:

CAGAACTCCA CACCCCCGAA G 21

(4) INFORMATION FOR SEQ ID NO: 3:

(i) SEQUENCE CHARACTERISTICS:

(A) LENGTH: 38
(B) TYPE: nucleic acid
(C) STRANDEDNESS: single
(D) TOPOLOGY: linear

(ii) SEQUENCE DESCRIPTION : SEQ ID NO: 3:

CCGCTAACCA CGACACTTTC TGTACTGCCT CTCAGCCG 38

(5) INFORMATION FOR SEQ ID NO: 4:

(i) SEQUENCE CHARACTERISTICS:

(A) LENGTH: 36
(B) TYPE: nucleic acid
(C) STRANDEDNESS: single
(D) TOPOLOGY: linear

(ii) SEQUENCE DESCRIPTION : SEQ ID NO: 4:

C[?][?]AACCCCG CACACACAAC CCCTACCCGG TTACCC 36

(6) INFORMATION FOR SEQ ID NO: 5:

(i) SEQUENCE CHARACTERISTICS:

(A) LENGTH: 45
(B) TYPE: nucleic acid
(C) STRANDEDNESS: single
(D) TOPOLOGY: linear

(ii) SEQUENCE DESCRIPTION : SEQ ID NO: 5:

TGATTCGTCA CGGGCGCCCA CACACGGGTA CGGGAATATC AACCC 45

(7) INFORMATION FOR SEQ ID NO: 6:

(i) SEQUENCE CHARACTERISTICS:

(A) LENGTH: 30
(B) TYPE: nucleic acid
(C) STRANDEDNESS: single
(D) TOPOLOGY: linear

(ii) SEQUENCE DESCRIPTION : SEQ ID NO: 6:

CTACTACCAG CCGAAGTTCC CACGCAGCCC 30

(8) INFORMATION FOR SEQ ID NO: 7:

(i) SEQUENCE CHARACTERISTICS:

(A) LENGTH: 37
(B) TYPE: nucleic acid
(C) STRANDEDNESS: single

(D) TOPOLOGY: linear

(ii) SEQUENCE DESCRIPTION : SEQ ID NO: 7:

GGAGTTGATC GATCCGGTTT TGGGTGGTTA GTACCGC 37

(9) INFORMATION FOR SEQ ID NO: 8:

(i) SEQUENCE CHARACTERISTICS:

(A) LENGTH: 43
(B) TYPE: nucleic acid
(C) STRANDEDNESS: single
(D) TOPOLOGY: linear

(ii) SEQUENCE DESCRIPTION : SEQ ID NO: 8:

GGGGTACGGG CCGTGTGTGT GCTCGCTAGA GGCTTTTCTT GGC 43